# EUROPEAN PATENT APPLICATION

(11) **EP 4 335 379 A1**
(43) Date of publication of application: **13.03.2024**
(21) Application number: 22194898.7
(22) Date of filing: 09.09.2022
(51) Int. Cl.: A61B 8/00

(54) **ULTRASOUND EXAMINATION DEVICE AND METHOD FOR OPERATING AN ULTRASOUND EXAMINATION DEVICE**

(71) Applicant: Siemens Healthcare GmbH, 91052 Erlangen (DE)
(72) Inventor: Heismann, Björn, 91058 Erlangen (DE); Niederdränk, Torsten, 85521 Ottobrunn (DE)
(74) Representative: Siemens Healthineers Patent Attorneys

(57) **Abstract**

Ultrasound examination device (1), comprising a transducer device (2) with a transducer (3) for emitting and receiving ultrasound, a user interface device (4) comprising an input device (7), and a control device (5) configured to control the transducer (3) based on user intended control information received from the input device (7), wherein the input device (7) comprises a microphone (9) for recording voice input data of a user, wherein the control device (5) is configured to derive the user intended control information from the voice input data.

## Description

The invention concerns an ultrasound examination device, comprising a transducer device with a transducer for emitting and receiving ultrasound, a user interface device comprising an input device, and a control device configured to control the transducer based on user-intended control information received from the input device. The invention further concerns a method for operating such an ultrasound examination device.

Ultrasound imaging is an established tool not only in medical diagnosis, but also in other areas. Ultrasound examination devices, in particular for ultrasound imaging, usually comprise a transducer device connected to a console acting as a user interface device. The ultrasound device comprises an ultrasound transducer, which can be placed against an object to be examined such that ultrasound may be emitted into the object to be examined and the reflected ultrasound may be received and measured to perform ultrasound imaging and/or measure certain properties, for example flow. The console usually comprises a monitor or display as an output device and an input device, in many cases a keyboard, where parameters can be set, imaging commands can be given and the like.

In medical applications, the console usually takes up a lot of space, is subject to hygiene regulations and is often difficult to handle, since the examiner/user both has to work with the usually hand-held transducer device and the input device.

During an ultrasound procedure, the examining person positions the ultrasound transducer device with the ultrasound transducer on the surface of the patient. By moving the transducer position and inclination, an imaging volume is selected. The console is used for choosing imaging properties, for example contrast, noise reduction, and the like, as well as imaging modes, for example normal ultrasound imaging or doppler blood velocity imaging. In most implementations, a display is sitting on top of the console or forms an integral part of the console. The resulting images may be shown combined with object and imaging information.

It is an object of the present invention to improve the operability and reduce the complexity of an ultrasound imaging device.

This object is achieved by providing an ultrasound examination device according to claim 1 and a method according to claim 15. Advantageous embodiments are described by the dependent claims.

In an ultrasound examination device as initially described, the input device comprises a microphone for recording voice input data of a user, wherein the control device is configured to derive the user intended control information from the voice input data.

Hence, the ultrasound examination device according to the present disclosure comprises a transducer device and a user interface device featuring voice-recognition capabilities, such that voice commands become available as an input mode. The currently used console unit with a display and button controls, in particular a keyboard, is no longer necessary. The input device of the current invention can be implemented in a cost-saving way and, in particular, also small, such that hand-held user input devices become feasible. Further, easy upgrade or retrofitting of existent devices, for example output devices such as monitors or projectors, is possible. The interactions between the user and the ultrasound examination device are, preferably predominantly, implemented by speech input. For example, the user can use their voice to change imaging parameters, for example by commands or sentences like "go doppler, blood color set" or "highlight vessels". In addition, commands relating to other functions are conceivable, for example regarding the display of acquired ultrasound images/data and the like. Here, modern, efficient speech analysis approaches can be employed to derive the user intention, even from different phrasing and wording. Hence, user intended control information can be reliably derived.

The operational complexity is greatly reduced. Less space-consuming operating controls and elements are required, in particular in highly crowded operational clinical environments. The user may change imaging parameters, imaging modes and the like without having to take their eyes from the object to be examined and the transducer device or a display device. Furthermore, the cost and complexity of the ultrasound examination device is reduced as a console is no longer necessary. The user interface device can be freely positioned within voice reach of the user.

In a concrete embodiment, the control device may comprise an automated speech recognition unit for determining natural language data from the voice input data and an evaluation unit for determining the user intended control information from the natural language data. Here, preferably, the evaluation unit may be a natural language processing unit. Automatic speech recognition (ASR) encompasses techniques that enable the recognition and translation of spoken language into text, in particular by using respective automatic speech recognition algorithms. Natural language processing (NLP) comprises techniques for analyzing natural language data in the sense of understanding the contents, in particular, in the current application, to derive respective user input commands as user intended control information from the natural language data. Hence, the evaluation unit may comprise at least one natural language processing algorithm.

Automated speech recognition (ASR) and natural language processing (NLP) have made significant progress over the recent years. A main reason is the development of BERT algorithms and their derivates. The acronym BERT stands for bidirectional encoder representations from transformers and is a transformer-based machine learning technique for natural language processing pre-training. Natural language processing algorithms enable artificial agents like control devices to understand the meaning of complex speech patterns. The current state of the art is capable of converting the audio input of a spoken sentence into text output for practically all relevant global languages, and natural language processing has reached a level of automizing the spoken tokens into relationships of meaning.

Hence, in the current invention, by using modern automated speech recognition techniques and natural language processing techniques, speech input can be processed naturally in the ultrasound examination device according to the invention. A highly intuitive interaction between the user and the device is possible. The true user intention can be derived as control information. In exemplary embodiments, the evaluation unit may be configured to use at least one trained function for natural language processing, in particular BERT implementation or a derivative therefrom.

For example, the control device may be configured to detect at least one cue word for addressing the ultrasound examination device in the voice input data and/or to determine user intended control information related to setting imaging parameters and/or steering the ultrasound beam of the transducer and/or related to questions regarding the ultrasound examination device, in particular help functions. Cue words may be used to identify spoken language as directed to the ultrasound examination device. Such a cue word may be, for example, a name of the device. Once the cue word is detected, for example in the natural language data, the control device decides that, in particular following, text is addressed to the ultrasound examination device.

In one embodiment, the user may also ask questions regarding the ultrasound examination device, for example invoking help functions and/or querying information, like "Which imaging mode are we in?", "What is the current value of imaging parameter X?" and the like. Regarding input commands relating to the ultrasound examination, voice can be used to steer the ultrasound beam up, down, left and right. Furthermore, the user can use his or her voice to change imaging parameters.

In some embodiments, it may be advantageous if the input device further comprises at least one manual operating element, in particular at least one button and/or wheel. Hence, some, in particular basic, functions may still be available via buttons or other manual operating elements, which may, at least partly, be provided at the user interface device. In preferred embodiments, however, at least one of the at least one manual operating element may be provided on the transducer device. Since the transducer device, which may also be called transducer head, is usually handled by the user anyway, for example by moving it across the surface of the object to be examined, such manual operating elements positioned on the transducer device can also easily be reached and actuated by the user, thus further simplifying operation of the ultrasound examination device.

Operating elements on the transducer device may, for example, comprise buttons for direct input of imaging parameters, for example for zooming in and out.

Preferably, the user interface device may further comprise an output device. The output device may comprise at least one audio device, in particular a speaker, wherein the control device comprises an output unit configured to determine and output at least one audio message related to the user intended control information-based control. In this manner, feedback information, like, for example, confirming receipt of an input command or answering a question posed to the ultrasound examination device, may also be audibly output such that there is no need for the user to turn his head to a visual output device, for example a display device, or the like. Hence, operating the ultrasound examination device is further simplified and a highly efficient work environment is provided.

Preferably, the output device may comprise at least one display device, wherein the output unit is configured to display ultrasound data acquired from the transducer on the display device. Such display devices (or, shortly, display) are already commonly used for ultrasound examination devices and are also expedient in a medical environment to also show acquired ultrasound data, in particular ultrasound images, to a patient to be examined.

Hence, regarding an ultrasound examination workflow, only slight changes regarding known devices are required. The user still examines the object, in particular the patient, using the transducer, while the display device may be positioned either behind or next to the patient or can be held in the second hand by the user. For inputting commands and the like, this second hand is not required since speech input is possible.

The ultrasound examination device may comprise communication means for wireless and/or wired communication between the transducer and the control device. That is, the control device, which may preferably be a part of the user interface device, and the transducer may communicate via a cable connection. However, it is preferred to at least partly use wireless communication, in particular a wireless local-area network (WLAN) or blue tooth technology. This reduces the number of wired connections required and increases flexibility.

In particular, according to the invention, the display device, where ultrasound data may be shown, is no longer tied to required operating elements of the input device, as was the case when using a console. Hence, the display device can be positioned where needed according to the workflow, for example in a position where both the user and a patient can view the results in some embodiments or in a position where only the patient or only the user has visual access, in particular if the patient is sedated or otherwise incapable of viewing the images.

As already mentioned, the functions of the control device may be at least partly, in particular completely, implemented in the user interface device. The control device may be integrated with the user interface device. In particular, the user interface device combined with the control device may also be configured for other applications, for example for controlling other medical devices or accessing information systems. Such a combined or integrated user interface device can be part pf the ultrasound examination device according to the invention, in particular avoiding the requirement of a custom-made user interface device.

In a particularly advantageous embodiment, the user interface device may comprise a hand-held, mobile device, in particular a tablet or a mobile phone. A commercially available mobile device, in particular a tablet and/or a mobile phone, for example a smartphone, may be configured to serve as user interface device with integrated control device for the transducer device. This reduces the cost and complexity of the ultrasound examination apparatus further since no custom-made user interface device and control device is required. In particular, such a mobile device may additionally be configured for access to further applications, for example to information systems or a picture archiving and communication system (PACS). Examples for such information systems comprise a hospital information system (HIS) or radiology information system (RIS). Furthermore, such a mobile device may also be configured to control other devices and applications. Hence, simplified integration into other medical systems is also provided. In particular, an "internet of medical systems" may be expanded. Further advantageously, such commercially available mobile devices already have a display, a microphone, a speaker and communication units integrated, which do not have to be additionally provided. They can be programed, such that software means may be provided to implement functional units, for example the mentioned automated speech recognition unit and evaluation unit.

In embodiments, the user interface device may be attached or attachable to the transducer device. For example, attachment means for a hand-held mobile device, in particular a tablet, may be provided opposite to the transducer, such that the impression of looking inside the object, in particular the patient, may be provided. In any case, by attaching the user interface device, in particular in the form of a hand-held mobile device, to the transducer device allows the user to keep all relevant components and the object in his field of view during the ultrasound examination.

In further embodiments, the user interface device may comprise an, in particular wall-mountable and/or flat-screen, monitor. In particular, any kind of TV or computer monitor may be used, wherein, for example, the microphone may be added. For example, if the display device is a flat-screen monitor, it may be mounted on a wall of an examination room wherein ultrasound examinations using the ultrasound examination device are performed.

In advantageous embodiments, the user interface device may also comprise an augmented and/or mixed reality device, in particular smartglasses, and/or an, in particular holographic, projector. That is, in one set-up, the user interface device may also comprise smartglasses or comparable augmented and/or mixed reality device. Smart glasses often also comprise microphones for detecting sound and speech. Information may be projected right into the view of the user. In other embodiments, the display device of the user interface device may be a holographic projector, to which an audio processing unit may be attached as input device to form the user interface device.

The invention also concerns a method for operating an ultrasound examination device, in particular according to the invention, wherein the ultrasound examination device comprises a transducer device comprising a transducer for emitting and receiving ultrasound, a user interface device comprising an input device, and a control device configured to control the transducer based on user intended control information received from the input device, wherein the input device comprises a microphone and wherein
- the microphone is used for recording voice input data of a user, and,
- by the control device, the user intended control information is derived from the voice input data.

All features and remarks regarding the ultrasound examination device may be analogously applied to the method according to the invention, such that the same advantages may be achieved. In particular, automated speech recognition and natural language processing may be applied.

Other objects and features of the present invention will become apparent from the following detailed description considered in conjunction with the accompanying drawings. The drawings, however, are only principle sketches designed solely for the purpose of illustration and do not limit the invention. The drawings show:
- Fig. 1: an embodiment of an ultrasound examination device according to the invention,
- Fig. 2: a close-up view of a transducer device with a user interface device attached,
- Fig. 3: further optional components of a user interface device, and
- Fig. 4: a flow chart of a method according to the invention.

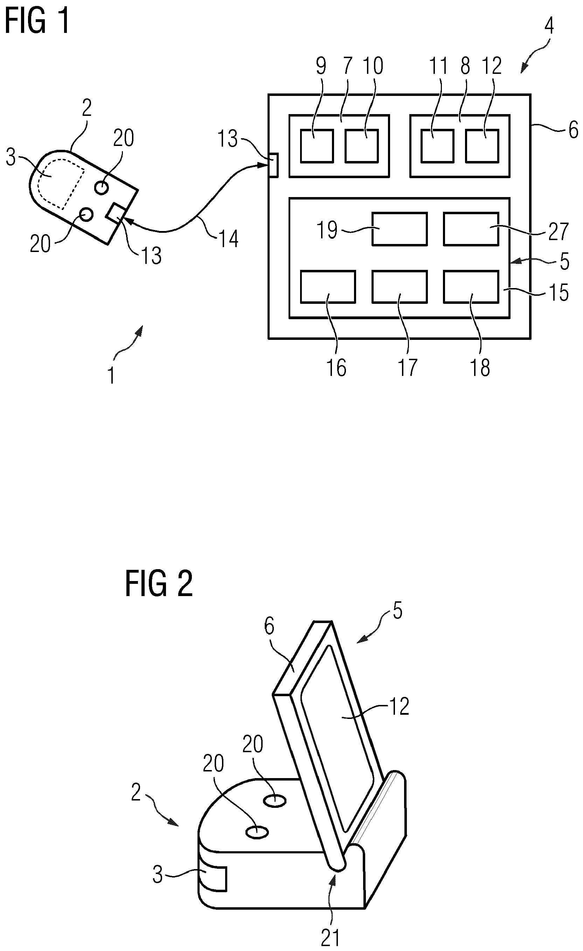

Fig. 1 is a principle drawing showing components of an ultrasound examination device 1 according to the invention. The ultrasound examination device 1 is a medical imaging ultrasound examination device. It comprises a hand-held ultrasound device 2. The ultrasound device 2 comprises an ultrasound transducer 3 for emitting and receiving ultrasound into an imaging region of a patient.

The ultrasound examination device 1 further comprises a user interface device 4 and a control device 5, wherein the control device 5 is, in this case, integrated into the user interface device 4. The user interface device 4 comprises a hand-held mobile device, in this case a tablet 6. As part of the user interface device 4, the tablet 6 provides components of an input device 7 and an output device 8, namely a microphone 9, at least one manual operating element 10, a speaker 11 and a display device 12, wherein at least a part of the at least one manual operating element 10 and the display device 12 may both be provided by a touch screen of the tablet 6. Communication means 13 provide a wireless communication link 14 between the transducer device 2 and the tablet 6, hence also the control device 5, which is implemented via at least one processor 15 of the tablet 6.

The control device 5 evaluates user input to the input device 7, that is, user intended control information (user input data), to control the transducer 3 based thereon. In particular, the control device 5 is configured to receive voice input data recorded using the microphone 9 and derive the user intended control information from the voice input data.

The voice input data is first analysed by an automated speech recognition unit 16 of the control device 5 to determine natural language data, in particular text representing the spoken language of the voice input data. This natural language data is further analysed by an evaluation unit 17 to determine the user intended control information from the natural language data. The evaluation unit is a natural language processing unit, which in particular uses at least one trained function to implement a natural language processing technique, for example employing a bidirectional encoder representations from transformers function.

In particular, at least one cue word may be detected, for example a name of the ultrasound examination device 1, indicating text addressed to the ultrasound examination device 1. From this text directed to the ultrasound examination device 1, input commands may be derived, for example input commands changing imaging parameters, input commands for steering the ultrasound beam and/or input commands requesting information, for example from implemented help functions and/or outputting further information. The derived input commands are forwarded to a control unit 18 executing these commands and also giving feedback via an output unit 27 using not only the display device 12, but also the speaker 11. In particular, an audio message acknowledging the receipt of input commands, providing requested information and/or giving help may be output via the speaker 11.

It is noted that the control device 5 may also comprise further functional units, for example ultrasound data processing unit 19 for evaluating measured ultrasound data and, for example, also using the output unit 27 for outputting respective evaluated ultrasound data, for example as an ultrasound image, on the display device 12.

In this case, a part of the input device 7 is also implemented on the transducer unit 2, which comprises at least two manual operating elements 20, for example for zooming in and out.

Fig. 2 shows a closer view onto the transducer device 2 having a transducer 3 and the operating means 20. In this case, the transducer device 2 further comprises an attachment means 21, which is only indicated in fig. 2, to attach the tablet 6 to the transducer device 2 such that, while using the transducer device 2, the user can also view ultrasound data or other information on the display device 12.

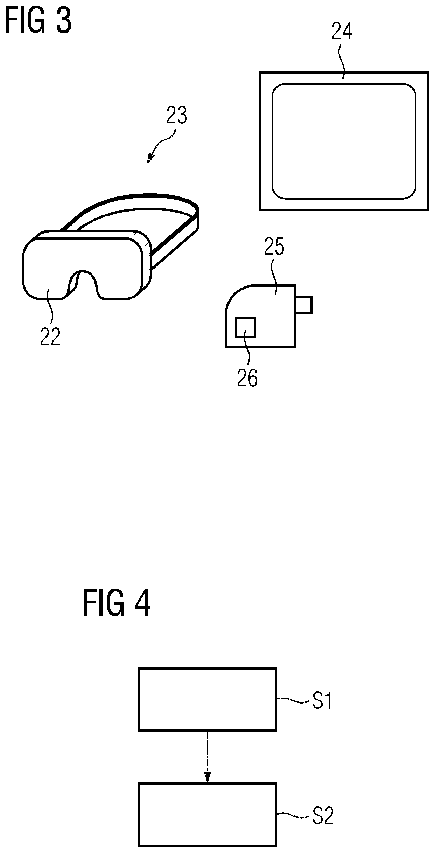

Fig. 3 shows optional further components of the user input device 4, namely smart glasses 22 as an augmented and/or mixed reality device 23, a wall-mountable monitor 24, for example a TV monitor, and a holographic projector 25 having an audio processing unit 26 attached thereon. Such components may be used alternatively or additionally to the preferred tablet 6, or generally, hand-held mobile device.

Finally, fig. 4 shows a general flowchart of a method according to the invention utilizing the ultrasound examination device 1 of figures 1 to 3. In a step S1, voice input data is recorded using the microphone 9. The voice input data is evaluated in step S2 by the control device 5 to derive user intended control information, in particular input commands. In step S2, in a first sub-step, automated speech recognition is applied to derive natural language data from the voice input data, and in a second sub-step, natural language processing is performed to derive the user intended control information.

Although the present invention has been described in detail with reference to the preferred embodiment, the present invention is not limited by the disclosed examples from which the skilled person is able to derive other variations without departing from the scope of the invention.

## Claims

1. Ultrasound examination device (1), comprising a transducer device (2) with a transducer (3) for emitting and receiving ultrasound, a user interface device (4) comprising an input device (7), and a control device (5) configured to control the transducer (3) based on user intended control information received from the input device (7), **characterized in that** the input device (7) comprises a microphone (9) for recording voice input data of a user, wherein the control device (5) is configured to derive the user intended control information from the voice input data.

2. Ultrasound examination device according to claim 1, wherein the control device (5) comprises an automated speech recognition unit (16) for determining natural language data from the voice input data and an evaluation unit (17) for determining the user intended control information from the natural language data.

3. Ultrasound examination device according to claim 2, **characterized in that** the evaluation unit (17) is a natural language processing unit.

4. Ultrasound examination device according to claim 3, **characterized in that** the evaluation unit (17) is configured to use at least one trained function for natural language processing, in particular a BERT implementation.

5. Ultrasound examination device according to one of the preceding claims, **characterized in that** the control device (5) is configured to detect at least one cue word for addressing the ultrasound examination device (1) in the voice input data and/or to determine user intended control information related to setting imaging parameters and/or steering the ultrasound beam of the transducer (3) and/or related to questions regarding the ultrasound examination device (1), in particular help functions.

6. Ultrasound examination device according to one of the preceding claims, **characterized in that** the input device (7) further comprises at least one manual operating element (10, 20), in particular at least one button and/or wheel.

7. Ultrasound examination device according to claim 6, **characterized in that** at least one of the at least one manual operating element (10, 20) is provided on the transducer device (3) .

8. Ultrasound examination device according to one of the preceding claims, **characterized in that** the user interface device (4) further comprises an output device (8).

9. Ultrasound examination device according to claim 8, **characterized in that** the output device (8) comprises at least one audio device, in particular a speaker (11), wherein the control device (5) comprises an output unit (27) configured to determine and output at least one audio message related to the user intended control information-based control, and/or at least one display device (12), wherein the output unit (27) is configured to display ultrasound data acquired from the transducer (3) on the display device (12).

10. Ultrasound examination device according to one of the preceding claims, **characterized in that** the ultrasound examination device (1) comprises communication means (13) for wireless, in particular WLAN and/or Bluetooth, and/or wired communication between the transducer (3) and the control device (5) .

11. Ultrasound examination device according to one of the preceding claims, **characterized in that** the control device (5) is at least partly, in particular completely, implemented in the user interface device (4).

12. Ultrasound examination device according to one of the preceding claims, **characterized in that** the user interface device (4) comprises a hand-held, mobile device, in particular a tablet (6), and/or is attached or attachable to the transducer device (2).

13. Ultrasound examination device according to one of the preceding claims, **characterized in that** the user interface device (4) comprises a wall-mountable and/or flat-screen monitor (24) .

14. Ultrasound examination device according to one of the preceding claims, **characterized in that** the user interface device comprises an augmented and/or mixed reality device (23) and/or a projector (25).

15. Method for operating an ultrasound examination device (1), in particular according to one of the preceding claims, wherein the ultrasound examination device (1) comprises a transducer device (2) comprising a transducer (3) for emitting and receiving ultrasound, a user interface device (4) comprising an input device (7), and a control device (5) configured to control the transducer (3) based on user intended control information received from the input device (7), **characterized in that** the input device (7) comprises a microphone (9), wherein
- the microphone (9) is used for recording voice input data of a user, and,
- by the control device (5), the user intended control information is derived from the voice input data.
